Europäisches Patentamt

European Patent Office

Office européen des brevets

(19)

(11) Publication number: **0 361 372**
**A1**

(12) # EUROPEAN PATENT APPLICATION

(21) Application number: **89117697.6**

(22) Date of filing: **25.09.89**

(51) Int. Cl.5: **C07C 57/05 , C07C 51/21**

(30) Priority: **26.09.88 US 249772**

(43) Date of publication of application:
**04.04.90 Bulletin 90/14**

(84) Designated Contracting States:
**AT BE CH DE ES FR GB GR IT LI NL SE**

(71) Applicant: **UNION CARBIDE CHEMICALS AND PLASTICS COMPANY INC. (a New York corporation)**
**39 Old Ridgebury Road**
**Danbury Connecticut 06817-0001(US)**

(72) Inventor: **Etzkorn, William George**
**5001 Ann Lee Drive**
**Cross Lanes West Virginia 25313(US)**
Inventor: **Harkreader, Gordon Gene**
**1506 Brentwood Road**
**Charleston West Virginia 25314(US)**

(74) Representative: **Weinhold, Peter, Dr. et al**
**Patentanwälte Dipl.-Ing. G. Dannenberg Dr. P. Weinhold Dr. D. Gudel Dipl.-Ing. S. Schubert Dr. P. Barz Siegfriedstrasse 8 D-8000 München 40(DE)**

(54) Anhydrous diluents for the isobutylene oxidation reaction to methacrolein and methacrolein oxidation to methacrylic acid.

(57) The processes for oxidation of isobutylene to methacrolein and the oxidation of isobutylene to methacrylic avoid in two stages with methacrolein as an intermediate are improved by use of anhydrous diluent gases to reduce or replace steam in the reaction streams. In particular, the use of anhydrous diluents which raise the composite flowing heat capacity of the diluent gas mixture to at least about 6.5 calories/gram-mole ($^{\circ}$C) will improve selectivity to desired products and will reduce the water load on the system.

FIG. 1

# ANHYDROUS DILUENTS FOR THE ISOBUTYLENE OXIDATION REACTION TO METHACROLEIN AND METHACROLEIN OXIDATION TO METHACRYLIC ACID

This invention relates to a process for the manufacture of methacrolein or methacrylic acid from isobutylene. More specifically, it describes an improved process for producing methacrolein or producing methacrylic acid by the catalytic vapor phase oxidation of isobutylene in the presence of inert, anhydrous diluents.

Generally, isobutylene in its gaseous phase is oxidized to methacrolein in the presence of molecular oxygen-containing gases and steam by contact at elevated temperatures with solid metal oxide catalysts. The methacrolein produced in this reaction stage can be recovered or can be directed without separation of the methacrolein to a second reactor operating in series with the first reactor to oxidize the methacrolein to methacrylic acid.

In the prior art, steam has been used in the starting reactant gas mixture in order to avoid flammable gas mixtures and because it was believed to be important to reaction selectivity. For example, US Patent No. 4,147,885 states that in a related process for oxidizing propylene to acrolein, it is wide practice to incorporate steam to avoid burning the reactant gases and increase selectivity to acrylic acid. Similarly, US Patent No. 3,475,48 discloses that it is desirable to incorporate steam in the starting reactant gas since this increases conversion and selectivity when employed in the order of 1 to 60, and preferably 5 to 30, moles of steam per mole of propylene or propylene + acrolein.

Other patents also describe steam as the preferred diluent. For example, US Patent 3,171,859 states that "addition of steam is obligatory ... it acts not only as a diluent, but also favors the reaction in that combustion to carbon oxides is substantially reduced." Also, US Patent 4,267,386 reiterates the general understanding among those skilled in the art, that while inert diluents may be added to the reaction system, "water, in the form of steam is desirably present ... in amounts of from 0.5 to 15, preferably 2 to 15, moles per mole of unsaturated hydrocarbon (i.e., propylene or acrolein)."

Many oxidation catalysts have been disclosed for producing methacrolein in high yield by oxidizing isobutylene. Predominantly, these are catalysts containing mixed oxides of molybdenum, bismuth and iron with phosphorous or tungsten or antimony. Cobalt and/or nickel and alkali metals are common promoters.

Catalysts which have been found to be advantageous for use in oxidizing methacrolein to methacrylic acid generally contain mixed metal oxides. Such catalysts typically contain molybdenum, vanadium, tungsten, chromium, copper, niobium, tantalum and antimony.

It is well recognized in the relevant catalyst art that water, in the form of steam, is an important component of the inert gas diluent (see, e.g., U.S. Patent No. 4,267,385, which states that it is "preferable to carry out the oxidation reaction in the presence of steam.") The ultimate object of the teachings in the literature cited above is to obtain high performance catalysts which give high selectivities to methacrolein and methacrylic acid at high isobutylene conversions. Other factors which influence the economic viability or the improved performance of these processes are not considered in these prior art techniques. For example, they do not adequately address the impact on process variables of use of high isobutylene concentrations, how to avoid the danger of explosion, the impact of inert reaction process feeds on recovery and waste disposal, or maintaining high catalyst performance over an extended catalyst life. These are all extremely important for commercial operation.

In commercial operation, it is of economic importance to minimize the presence of the steam which is fed to the reactors, since it passes through the system and becomes a burdensome wastewater load after product recovery steps; nevertheless, to the knowledge of the present inventors, no commercial process has been successfully operated below a steam: isobutylene mole ratio of about 1.5. Furthermore, it is extremely important to minimize by-products which are difficult to separate from useful product or which carry a high economic penalty for disposal. process improvements which will provide high catalyst performance while simultaneously maximizing isobutylene feedstock usage, and improvements which may promote conditions for extended useful catalyst life are important for commercial operation. The prior art does not adequately address these issues.

US Patent 4,049,577 teaches an improved catalyst composition for making acrolein. The authors mention that recycle gas comprised of the noncondensable fraction of the product can be used in place of steam. They suggest that these recycled inerts are preferable to steam as diluent since they allow higher conversions of propylene and thus enable one to obtain higher yields, and also reduce the water load on the system; however, the use of recycled inerts is stated as being made possible by the characteristics of this particular catalyst composition. Nowhere does this patent suggest or teach that anhydrous diluents have an improved effect on selectivity or product mix, or are useful with other catalysts. The relationships between various diluents and the heat capacity effects on selectivity are not suggested.

US Patent 3,801,634 teaches the use of inert solids mixed with active catalyst in the first and second stage reactors used to manufacture acrolein and acrylic acid. The authors indicate that noncondensable, second-stage effluent gases can be recycled to the first stage as inert diluting gas which can, at least partly, replace steam. The authors do not show any relationship between the inert anhydrous diluent gases and improvements in product selectivity.

US Patent 4,031,135 presents a recycle process in which noncondensable gases, preferably including steam, are recycled to the first-stage reactor and also to the interstage (second-stage) reactor feed. There is no recognition of the benefits in using anhydrous diluents with respect to their effect on by-product selectivity mix. The benefits in using anhydrous diluents with respect to their effect on by-product selectivity mix. The authors do, however, recognize an apparent improved acrylic acid efficiency, which they attribute partly to the use of inert diluents other than steam. They do not prove that this is true, nor do they show the effect of heat capacity on product selectivities.

US Patent 4,365,087 refers to the recycling of dewatered residue gas, containing both inert and reactive gases, to increase the concentration of acrylic acid recovered. However, the authors consider this procedure unsatisfactory since the composition of the residue gas fluctuates.

US Patent 4,442,308 teaches the use of inert gases as diluent in the acrolein process; however, it specifies their use for a particular supported first-stage acrolein catalyst. Most common commercial catalysts for propylene oxidation to acrolein are neat (unsupported) and do not follow this patent's prescribed preparation. This patent also claims that 0.5 to 7 mole % steam is beneficial and recommended. Nowhere in this patent do the authors teach the advantage of anhydrous diluents on product mix nor do they mention flowing heat capacity as a major variable in controlling product selectivity to advantage.

US Patent 4,456,006 teaches a catalyst preparation for the propylene-to-acrolein reaction. It shows that nitrogen diluent presents an improvement over steam diluent when used with this catalyst. It does not teach the heat capacity effect of diluent on product selectivity, nor does it show by-product reductions when using anhydrous diluents.

US Patent 3,717,675 describes a process for recovery of acrylic acid where acrolein is expelled from the aqueous acid collected and returned to the reactors to increase subsequent yields of acrylic acid. This patent mentions the use of inert diluents such as carbon oxides and nitrogen, but does nothing to demonstrate their importance. In fact, it states that it is necessary to add steam to the reaction in order to increase selectivity.

UK Patent application No. 2,068,947 teaches a process for producing methacrolein and methacrylic acid whereby inert anhydrous diluent gases are used, also combined with water vapor to produce a product with a reduced quantity of condensables compared to the typical steam diluent process. The authors fail to recognize the relationship between anhydrous diluents and acetic acid reduction, and they do not address selectivity improvements resultant from use of various anhydrous diluents.

US Patent 4,147,885 describes a recycle process in which steam is an essential ingredient. The object of the patented invention is to recycle steam to the reactors. This is contrary to the techniques of the instant invention, since it has now been found that the reduction or absence of steam is beneficial.

None of the prior art recognizes the use of various inert anhydrous diluents in specific proportions so as to favorably affect the product mix obtained over any of the commonly used catalysts.

As has been indicated above, the basic two-stage process for oxidizing isobutylene to methacrylic acid via methacrolein is well known and has been described in the literature. In connection with the related acrylic acid process, it is also known that wet, overhead gases (noncondensables) from the acrylic acid scrubber can be recycled to the first reactor stage. By this recycling of unreacted propylene and acrolein, it is predictable that an improvement in overall yield is obtained. By use of such a recycle stream, it is also possible to provide a supplemental means of controlling the steam content to the first-stage reactor, as is taught in U.S. Patent No. 4,147,885. In the process of that patent, the steam content of the first-stage feed is required to be 4 to 30% by volume, with all the steam, except that in the starting reactant gas mixture, being provided by the recycle stream. As discussed above, however, the presence of even as little as 4% steam is disadvantageous.

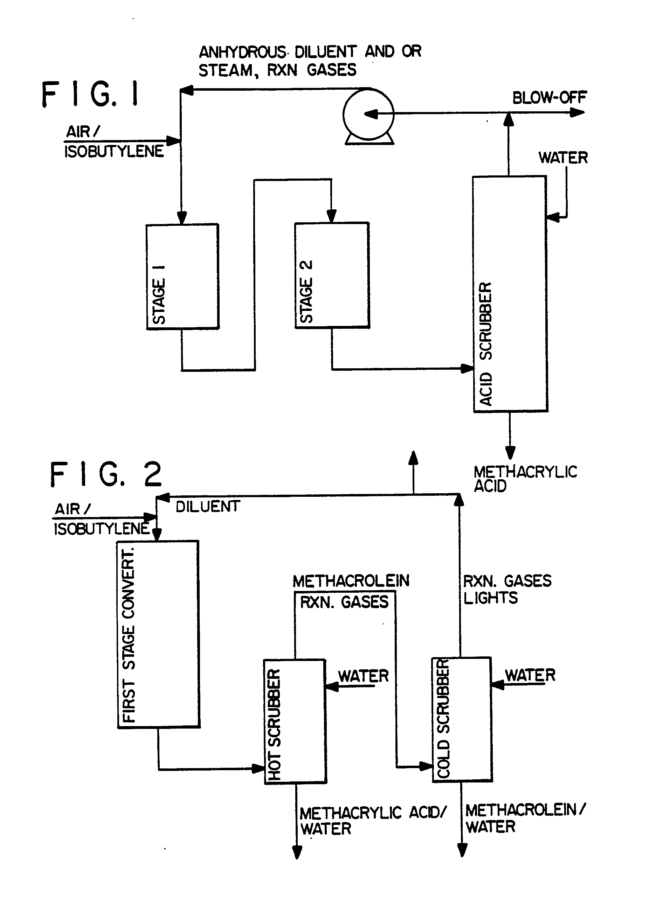

Figures 1 and 2 illustrate the application of recycle streams to methacrolein and methacrylic acid processes.

The present invention embraces two separate but related concepts, namely the reduction or elimination of steam to reduce the water load on the process, and the improvement of selectivity to maximize the output of desired products. Both of these results are achieved by replacement of some or all of the steam diluent customarily used in prior art processes with an anhydrous diluent (optionally containing a minimal amount of steam) having a heat capacity in a selected range.

In a preferred embodiment, a portion of the non-condensable gases from the process, e.g., the overhead stream from the acid scrubber, is recycled back to the first-stage reactor feed stream to replace at least some of the steam in that stream.

It will be understood that the process of this invention can be applied not only to a combined isobutylene methacrolein-methacrylic acid process, but also to a separate methacrolein-methacrylic acid process, or to the methacrolein-methacrylic acid leg of an isobutylene methacrylic acid process. Thus, a portion of the stream from the first-stage isobutylene methacrolein reactor can be sent to a methacrolein recovery process, from which some or all of the non-condensable overhead gases from the methacrolein scrubber system can be recycled as diluent to the first and/or second stage of the isobutylene methacrylic acid process.

For purposes of this invention, the term "isobutylene" is intended to include as well other compounds which can be converted in situ to isobutylene, e.g., t-butyl alcohol, t-butyl acetate, methyl t-butyl ether, and the like, and/or which respond to the same catalysts as does isobutylene and proceed through the same intermediates (i.e., iso-butylene and methacrolein).

According to the invention, it has been discovered that anhydrous diluent gases can be used to reduce or completely replace steam in the isobutylene oxidation reaction to efficiently produce methacrolein and methacrylic acid. (For purposes of this invention, a diluent is any gas which does not react in the reaction stage in which it exists.) Furthermore, when replacing the steam diluent with anhydrous diluents, major by-products, e.g., acetaldehyde and acetic acid, are significantly reduced. These reductions in by-products are especially important since acetaldehyde forms acetic acid over the methacrolein-to-methacrylic acid reaction step, and acetic acid is a significant waste product. To make saleable quality methacrylic acid, considerable energy is required to more completely remove the acetic acid. Waste disposal costs for disposing of acetic acid are high. The present invention provides a means for reducing acetic acid disposal costs, decreasing separation costs for both methacrolein recovery and methacrylic acid recovery, and enables existing equipment to enact a better separation, thus saving methacrylic acid recovery losses and providing potential for higher quality refined product.

Another key discovery of this invention is that by increasing the flowing heat capacity of the reactant gas mixture, the yield of useful products can be increased significantly. The heat capacity is increased by the introduction of an anhydrous diluent having a relatively high composite heat capacity (as defined herein), comprising one or more gases with relatively high molar heat capacities. Flowing heat capacity is the composite heat capacity of the anhydrous diluent, plus the heat capacity of the reactants, i.e., the composite heat capacity of the total gas stream. However, this does not change appreciably as a result of reaction, since various reaction products have a higher heat capacity than that of the reactants, and some have a lower heat capacity. In general, the flowing heat capacity will not be expected to change by more than about one heat capacity unit as a result of reactions. Thus, the composite heat capacity of the diluent is a dominant variable for process control purposes.

As the flowing heat capacity of the reaction feed gas mixture is increased, yield to methacrolein, and methacrolein + methacrylic acid increase, and the flammable gas range is reduced, enabling higher productivity operations. Simultaneously, the peak temperature in the catalyst bed due to the exothermic heat of reaction is lessened and the heat of reaction that is released is absorbed more efficiently in the bulk gas stream. This, in turn, should increase catalyst life by decreasing thermal stresses within the catalyst pellets' structure, reducing potential carbon build-up within catalyst pores, and by reducing pressure drop, since there will be lower volumetric flow of reactant gas feeds necessary to meet a given production level.

The invention is advantageous for recycling diluent gases and unreacted isobutylene back to the reactors. The resulting low-steam-containing product streams provide an ample source of noncondensable diluent, so that separation of useful product is simplified. This is particularly advantageous for methacrolein recovery, since methacrolein, which is more volatile than water, can be effectively separated from the reaction-produced water without loss of diluent. By using anhydrous diluents with higher volatilities compared to methacrolein, the present invention permits operation on a methacrolein, recovery system with recycle of diluent, unreacted isobutylene, and unrecovered methacrolein back to the reactor for further efficiency gains and cost reductions. Such a system using steam diluent is not possible when adapting prior art methacrolein recovery equipment and techniques. It also enables implementation of recycle processes in which components such as acetic acid and acrylic acid and other minor, heavy by-products are excluded from the recycle stream. This is significant since the acids and heavy by-products are suspected of adversely affecting catalyst life, and furthermore, it aids in minimizing recycle handling problems, such as compressor corrosion.

The composition of the process feeds must be comprised so that flammable gas mixtures are not formed. According to this invention, the starting reactant gas mixture typically contains up to about 16 g-moles per hr. of isobutylene, preferably up to about 8 g-moles per hr. of isobutylene, per liter of first-stage catalyst; about 1.1 to about 2.2 moles of molecular oxygen per mole of isobutylene, and an inert diluent gas which comprises about 40 to about 94% by volume of the feed stream. It is desirable that the mole ratio of composite diluent to isobutylene be in the range of about 2 to about 32. The diluent gas typically comprises a mixture of one or more of nitrogen, carbon dioxide, methane, ethane, propane, butanes and steam; however, any other inert gas can be included. Some other useful inert gases include helium, argon, saturated hydrocarbon gases, $N_2O$, and carbon monoxide. When used, the quantity of steam in this invention should be no more than about 0.4 mole per mole of isobutylene, preferably 0 to about 0.3 mole per mole of isobutylene. The inert diluent should be of sufficient quantity to avoid flammable mixtures when combined with the isobutylene and molecular oxygen. Air or oxygen can be used as the molecular oxygen source. Of course, if air is used, the contained nitrogen acts as a supplemental diluent.

For each diluent gas mixture there is a relationship which can be determined by experiment and which describes the limiting compositions of oxygen, isobutylene, and diluent for which flammable mixtures exist. Most commercial applications are most desirably operated in a "fuel-rich" mode, whereby the oxygen content is the limiting factor from a flammability standpoint. The isobutylene concentrations will be determined by catalyst performance and by commercial cost effectiveness factors.

It is a distinct advantage of this invention that, since diluent gas mixtures with high composite heat capacities have a tendency to broaden the operable range due to shrinkage of the flammable gas envelope, high isobutylene concentrations are possible. It is theorized that first stage isobutylene feed concentrations as high as about 30 mole % will be achievable using the method of this invention.

Approximate ranges for feed compositions are defined based on the generalized operating constraints discussed above. First-stage feeds in the following ranges are particularly useful:

Isobutylene: 0 to 16 g-mole per hr. per liter of first-stage catalyst, preferably 0 to 8 g-mole per hr. per liter of first-stage catalyst;

Oxygen: 1.1 to 2.2 $O_2/C_4H_8$ ratio, such that there is 0 to 33.6 g-mole $O_2$ per hr. per liter of first-stage catalyst, preferably 0 to 21 g-mole $O_2$ per hr. liter of first-stage catalyst;

Diluent: 2.0 to 32 diluent/$C_4H_8$ ratio, preferably 3.5 to 12 diluent/$C_4H_8$ ratio.

The process of the invention is particularly advantageous in that it is not dependent upon any particular catalyst, as is much of the prior art, and will provide its benefits for any catalyst of choice. Any molybdenum, iron-based mixed metal oxide catalyst, such as those disclosed in U.S. Patents 3,825,600; 3,649,930; 4,339,355; 4,267,385; and 4,306,090; (catalysts relevant to the first stage are also shown in U.S. Patents 4,012,449 and 4,354,044) can be used in the isobutylene-to-methacrolein oxidation reactor. A Mo, P-based mixed metal oxide catalyst, such as described in U.S. patents 4,419,270; 4,444,907; and 4,051,179 can be used effectively in the second stage (i.e., the methacrolein oxidation to methacrylic acid reaction).

The general reaction conditions are not narrowly critical, and are those known to the art. The first-stage reaction operates at temperatures of 250°C to about 450°C, although temperatures of about 300°C to about 400°C are preferred. The second-stage reaction requires temperatures of about 200°C to about 450°C, with a preferred range of about 250°C to about 375°C.

Operating pressures of about 1 to about 4 atmospheres are typical, although this process improvement will apply for all operating pressures, whether subatmospheric, atmospheric, or superatmospheric. Preferred commercial modes of operation will minimize pressures, but pressures are typically held in the 2 to 3 atm range due to system pressure-drop constraints.

Flow rates can be varied from about 0.5 to about 15 seconds contact time; however, typical commercial flow provides about 1.5 to about 4.0 seconds contact time. Contact times of about 1.7 to about 3.0 seconds are preferred.

As indicated above, selection of proper heat capacity of the anhydrous diluent gas or gases is critical to the proper performance of the invention. Since the diluent gas stream may comprise a mixture of several individual gases, it is convenient to refer to a composite heat capacity for the total stream. The term "composite heat capacity," as used herein, means the sum of the products of the volumetric fraction of each gas in the diluent gas mixture and its heat capacity. (Heat capacity, as referred to herein, is the ideal gas heat capacity determined at 330°C for purposes of the composite heat capacity definition.) The composite heat capacity for the diluent gas going to the first stage reactor should be at least about 6.5 calories/gram-mole (°C). Below this value, the product selectivity benefits of this invention are minimal. There is no known upper limit on composite heat capacity; however, it is theorized that above a value of about 40 there may be an unrecoverable heat loss through absorption of reaction heat into the process stream, which would result in an economic penalty. In addition, there could be a problem with increased after-burning at the exit of the first-stage reactor. It is preferred that the composite heat capacity be maintained in the range of about 8 to about 30, and most preferably about 10 to about 20.

5

The flowing heat capacity of the second-stage reactor feed gases is determined predominantly by the choice of anhydrous diluent gas fed to the first-stage reactor. The first-stage product mix has only a minor influence on the second-stage feed flowing heat capacity, since the products account for only about ten to twenty percent of the total stream volume. For example, a typical operation with 6% isobutylene and 13% oxygen produces water plus methacrolein, methacrylic acid, acrolein, acrylic acid, acetaldehyde, acetic acid, and carbon oxides. The average heat capacity of the feed isobutylene and oxygen is very nearly the same as the average heat capacity of the resulting products (approximately 0.65 cal/g-mole ($0^{\circ}$ C) more for products versus reactants).

The anhydrous diluent gas of this invention can be a single gas or a multi-component mixture of gases, provided that certain criteria are observed. Each gas must be inert to the oxidation reactions of the process, and each gas must be noncondensable and readily separable from the reaction products.

Since each plant installation will have specific constraints that affect the energy usage for the entire plant, attention must be paid to the impact on the plant energy balance for use of particular diluents which alter the current heat recovery schemes. For example, a high heat capacity diluent will retain more of the heat evolved through reaction, whereas now the bath temperature is relied on more to remove and recover the heat of reaction. High heat capacity diluents will require more attention to recovering heat after the reaction. Furthermore, if process off-gas is disposed of via combustion, the recovery of heat will be affected by a major change in diluents.

In addition, one should avoid catalyst poisons, e.g., sulfur dioxide, and gases that react to unwanted by-products, as would unsaturated hydrocarbon compounds (e.g., propylene), or $NH_3$, which produces methacrylonitrile.

It is another advantage of this invention that the steam component typically contained in the feed to the first stage can be minimized, even eliminated. While there is controversy among those skilled in the art as to the precise function of steam, e.g., whether it is truly an inert diluent or whether it somehow participates in the oxidation of isobutylene and methacrolein, it is accepted practice in the art that a significant concentration of steam is required in order to successfully operate the first-stage and second-stage reactions. Contrary to this holding of the art, it is surprising discovery of the present invention that steam is desirably minimized and can be eliminated entirely. This is accomplished by substituting for the steam the inert gas diluent of selected composite heat capacity of this invention. Accordingly, it has been found that the steam content of the feed gas can be essentially zero. While not preferred, the steam content of the feed gas can range as high as about 3% by volume of the feed gas. It is preferred that the steam content of the feed stream be kept below about 2% more preferably below about 1%, by volume.

While not an absolute requirement of this invention, it is highly preferred that the inert diluent gas used be, at least in part, an essentially anhydrous recycle stream from within the process. Preferably, this will comprise a portion of the noncondensable, overhead gas mixture from the methacrolein or methacrylic acid recovery scrubber train which removes water and methacrylic acid from the product mixture. In particular, the use of low-boiling, anhydrous diluents makes recycle from a methacrolein recovery process possible. Besides providing a beneficial system which allows recovery of current methacrolein separation efficiency losses and reuse of unconverted isobutylene, recycling process gases from a methacrolein recovery process is desirable and advantageous to recycling process gases from a methacrylic acid recovery process as described in the prior art.

In order to minimize the water vapor carried overhead from the scrubbers, they should be operated within the ranges of conditions shown in Table I.

## Table I

| | METHACRYLIC ACID SCRUBBERS: (Methacrolein Recovery System or Methacrylic Acid Recovery System | METHACROLEIN SCRUBBER: (Methacrolein Recovery System) |
|---|---|---|
| BASE TEMP. (°C) | <95, preferably: 80 | <45, pref.: 15 to 35 |
| HEAD TEMP. (°C) | <80, pref.: <70 most. pref.: <60 | <40, pref.: 10 to 30 |
| PRESSURE (ATM) | <3, pref. 1. to 2. | <3, pref.: 1. to 2. |
| $\dfrac{\text{SCRUBBING MEDIUM FLOW (Volume)}}{\text{BOTTOM PRODUCT STREAM FLOW (Volume)}}$ | <1/1 pref.: <1/2. | |
| $\dfrac{\text{SCRUBBING MEDIUM FLOW (Weight)}}{\text{ACROLEIN BOTTOMS FLOW (Weight)}}$ | <80/1 pref.: <30/1 | |

Under most operating conditions, it will be necessary to take off a purge stream, the size and location of which will be determined by the specific process being used. If pure oxygen is used as the source of oxygen, the purge can be relatively small. If air is used as the oxygen source, there will be a build-up of inerts, e.g., nitrogen, so that a substantial purge will be required, and will be controlled to maintain the desired composite heat capacity. The use of pure oxygen (i.e., oxygen not burdened with substantial concentrations of inert gases) permits maximization of diluents with a heat capacity higher than that of nitrogen. This permits minimization of the purge, which in turn, makes feasible the use of high heat capacity gases, such as propane or butane, which might be too expensive for use as diluents if they were to be substantially lost through purging.

## EXAMPLES

The examples which follow illustrate the invention, but are not intended to limit it in any way. In these examples, all concentrations are in mole percent.

## Example 1

A pilot plant experimental set-up consists of two single tubular reaction vessels of typical commercial reactor tube dimensions. The first reactor tube contains a commercial catalyst comprising molybdenum bismuth, iron and several promoter metals typical of first-stage catalyst, as described above. The second reactor tube is filled with a commercial second-stage catalyst, similar to those described previously, and is connected in series with the first. The gaseous reaction products are sampled and separated into condensable and noncondensable portions. Each phase sample is measured and analyzed by gas chromatograph. The resultant measurements are used to calculate reaction yields and isobutylene conversions. These sampling procedures are accomplished for both first-stage product and second-stage product, so that process performance for methacrolein production and process performance for methacrylic acid production are both determined. A jacket surrounding each tube is filled with a heat transfer fluid which circulates to remove heat of reaction. Thermocouple and sample taps are provided along the length of each reactor and at the bottom of each reactor. Gas feeds are metered into the first reactor using mass flowmeters. The first-stage effluent is then conducted directly into the second-stage reactor. The condensable portion of the second-stage effluent is recovered as a liquid tails streams from a water-based scrubber. Non-condensable gases are conducted out the top of the scrubber (and could be returned, if desired, to the reactors to supply additional diluent gas). The system outlet pressure is controlled at 7 psig in order to control reactor feed pressures on the system. Isobutylene feed concentration is set at 6.0% and air feed concentration is set at 60.2%. The additional (to nitrogen in the air feed) feed gas diluent contains 2.6% nitrogen and 30% steam. (Also present is about 0.2% inert impurities in the isobutylene.) The system outlet pressure is set at 7 psig, and reactor temperature is set to 350° C.

A typical isobutylene conversion of 98% with 85% selectivity to methacrolein plus methacrylic acid is achieved.

## Example 2

Example 1 is repeated but with 32.6% nitrogen and 0% steam as the diluent gas. Temperature is adjusted to give a first-stage isobutylene conversion of 98%.

A reduction of about 50% in acetaldehyde and acetic acid byproducts is observable. Methacrolein plus methacrylic acid selectivity is increased by about 1%.

## Example 3

The conditions of Examples 2 are repeated with methane instead of nitrogen in the diluent gas, the remainder being steam.

Acetaldehyde and acetic acid byproduct selectivities are reduced by about 50%. Methacrolein plus methacrylic acid selectivity increases by about 3%.

The terms "conversion," "yield," "selectivity," "space velocity," and "contract time" are defined as follows:

$$\text{conversion \%} = \frac{\text{moles isobutylene converted}}{\text{moles isobutylene fed}} \times 100$$

$$\text{yield (mole\%)} = \frac{\text{moles product produced}}{\text{moles isobutylene fed}} \times \frac{\text{number carbon atoms in product}}{4} \times 100$$

$$\text{selectivity (mole\%) b} = \frac{\text{moles product produced}}{\text{moles isobutylene converted}} \times \frac{\text{number carbon atoms in product}}{4} \times 100$$

$$\text{space velocity (hr}^{-1}) = \frac{\text{gas volumetric flowrate (1/hr)}^*}{\text{volume of reactor catalyst bed (1)}}$$

$$\text{contact time (seconds)} = \frac{3600}{\text{space velocity}}$$

*Flow adjusted to standard temperature and pressure (i.e., 0°C and 1 atm.)

Recycle Applications

Recycling of process streams is well known in the chemical process arts, and is usually implemented to improve reaction efficiencies and process economics. More specifically, recycling of product or a portion of a product stream enables efficient use of feed material not reacted in a single pass or reuse of feed material which is costly to make up in the reactor feed stream. Use of anhydrous diluents has a particularly advantageous effect on the operability of recycle. It enables using a recycle stream which has less acid, thus increasing compressor operability. Furthermore, prior art recycle processes require more elaborate sampling mechanisms in order to reliability measure recycled oxygen concentrations. The control of oxygen is essential to safe operation of these recycle processes due to concerns over flammable gas mixtures. The anhydrous streams of this invention, however provide for reliable and accurate monitoring of oxygen, thereby increasing the recycle process reliability and operability as well as safety.

Furthermore, an anhydrous diluent process allows simple, efficient recovery and recycle of methacrolein in a methacrolein production unit.

**Claims**

1. Process for producing methacrylic acid by a two-stage catalytic oxidation of isobutylene, wherein the first stage produces primarily methacrolein and the second stage produces primarily methacrylic acid by oxidation of methacrolein, said process utilizing one or more recycle streams to either or both stages, both stages operating on feed streams containing oxygen and an inert diluent gas, characterized by utilizing as the diluent gas to the first stage a gas mixture comprising about 0 to about 0.4 mole of steam per mole of isobutylene, the remainder of the diluent gas comprising one or more inert gases such that the diluent gas has a composite heat capacity of at least about 6.5 calories/gram-mole ($^{\circ}$C) and utilizing as the diluent gas to the second stage a gas comprising one or more inert gases having a composite heat capacity of at least about 6.5 calories/gram-mole ($^{\circ}$C).

2. A process of claim 1 wherein the composite heat capacity of the inert diluent gas to the first stage is about 6.5 to about 40.

3. A process of claim 2 wherein the composite heat capacity of the inert diluent gas to the first stage is about 8 to about 30.

4. A process of claim 3 wherein the composite heat capacity of the inert diluent gas to the first stage is about 10 to about 20.

5. A process of any one of claims 1 to 4 wherein the steam content of the diluent gas to the first stage is less than about 0.4 mole per mole of isobutylene.

6. A process of claim 5 wherein the steam content of the diluent gas to the first stage is less than about 0.3 mole per mole of isobutylene.

7. A process of any one of claims 1 to 6 wherein the oxygen is from a pure oxygen source.

8. A process of any one of claims 1 to 7 wherein the diluent gas comprises a recycled process stream from a methacrolein recovery operation.

9. A process of any one of claims 1 to 8 wherein the diluent gas comprises a recycled process stream from a methacrylic acid recovery operation.

# FIG. 1

ANHYDROUS DILUENT AND OR
STEAM, RXN GASES

BLOW-OFF

AIR /
ISOBUTYLENE

WATER

STAGE 1

STAGE 2

ACID SCRUBBER

METHACRYLIC
ACID

# FIG. 2

AIR /
ISOBUTYLENE

DILUENT

FIRST STAGE CONVERT.

METHACROLEIN
RXN. GASES

RXN. GASES
LIGHTS

HOT SCRUBBER

WATER

COLD SCRUBBER

WATER

METHACRYLIC ACID/
WATER

METHACROLEIN /
WATER

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| X | EP - A2 - 0 253 409 (UNION CARBIDE) * Claims * | 1-9 | C 07 C 57/05 C 07 C 51/21 |
| A | EP - A1 - 0 274 681 (MITSUBISHI) * Example; claims * | 1-4, 7-9 | |
| P,A | US - A - 4 267 385 (S. UMEMURA) * Claims * | 1-4 | |
| A | DE - A1 - 2 436 818 (BASF) * Examples; claim * | 1-4,8, 9 | |
| D | & US-A-4 031 135 | | |
| A | GB - A - 1 539 671 (NIPPON SHOKUBAI KAGAKU KOGYO) * Claims * | 1 | |

TECHNICAL FIELDS
SEARCHED (Int. Cl 4)

C 07 C 57/00

The present search report has been drawn up for all claims

| Place of search | Date of completion of the search | Examiner |
|---|---|---|
| VIENNA | 11-12-1989 | HOFBAUER |